(19) Europäisches Patentamt
European Patent Office
Office européen des brevets

(11) EP 1 238 668 A1

(12) **EUROPEAN PATENT APPLICATION**

(43) Date of publication:
**11.09.2002 Bulletin 2002/37**

(51) Int Cl.[7]: **A61K 31/407**, A61P 25/00

(21) Application number: **01105959.9**

(22) Date of filing: **09.03.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Aventis Pharma Deutschland GmbH**
**65929 Frankfurt am Main (DE)**

(72) Inventors:
- **Eder, Claudia Dr.**
  **65719 Hofheim a. TS. (DE)**
- **Kurz, Michael Dr.**
  **65719 Hofheim a. TS. (DE)**
- **Wink, Joachim Dr.**
  **63322 Rödermark (DE)**

Remarks:
The biological material has been deposited with Deutsche Sammlung von Mikroorganismen und Zellkulturen GmbH (DSMZ), Mascheroderweg 1b, D-38124 Braunschweig under number(s) DSM 13834

(54) **Use of thiolutin dioxide and its derivatives in the manufacture of a medicament for the treatment of CNS disorders and a process for the preparation thereof**

(57) The present invention relates to the use of thiolutin dioxide and its derivatives in the manufacture of a medicament for the treatment of CNS disorders, to a process for the production thereof by fermentation of the microorganism Nocardiopsis species ST 100692, DSM 13834 , and to the micro-organism Nocardiopsis species ST 100692, DSM 13834.

EP 1 238 668 A1

## Description

The present invention relates to the use of thiolutin dioxide and its derivatives in the manufacture of a medicament for the treatment of CNS disorders, to a process for the production thereof by fermentation of the microorganism Nocardiopsis species ST 100692, DSM 13834, and to the micro-organism Nocardiopsis species ST 100692, DSM 13834.

Thiolutin is a known natural compound which is available commercially (Apin Chemicals, UK; CMS Speciality Chemicals, UK; Ubichem plc, UK). Thiolutin dioxide is also a known compound. One known process for the preparation of thiolutin dioxide is by oxidation of thiolutin with m-chlorperbenzoic acid (Yield 30 %) as described in Schachtner et al., J. Heterocycl. Chem., 1999, 161-175.

Thiolutin dioxide has previously been described as having medicinal properties. WO 99/12543 describes the use of thiolutin dioxide as an antineoplastic agent and WO 96/32396 describes the use of thiolutin dioxide as an antibacterial and antifungal agent.

It has now surprisingly been found that thiolutin dioxide is an effective inhibitor of neurolysin. Neurolysin belongs to the family of zinc-containing metalloproteases. It plays a likely role in the physiological inactivation of neurotensin, an endogenous antipsychotic agent. Thiolutin dioxide inhibits the neurotensin inactivation of neurolysin and is therefore useful in the treatment of neurodegenerative diseases such as Parkinson's and Alzheimer's. Thiolutin dioxide is also selective in that it does not block other zinc-containing metalloproteases such as enkephalinase or angiotensin converting enzymes.

It has now also been found that the microorgansim Nocardiopsis species ST 100692, DSM 13834, is able to form thiolutin dioxide in relatively high yield.

The present invention accordingly relates to the use of a compound of formula I

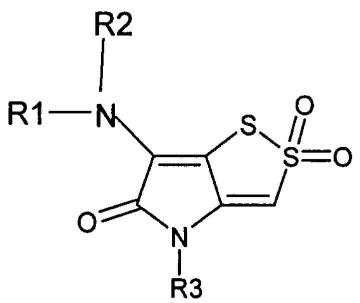

I

wherein

$R_1$, $R_2$ and $R_3$ are independently selected from: H, alkyl, and acyl, and physiologically tolerated salts thereof, in the manufacture of a medicament for the treatment of CNS disorders.

Preference is given to compounds of formula I in which $R_1$ is acyl, $R_2$ is H and/or $R_3$ is alkyl; and the physiologically tolerated salts thereof.

The acyl radicals in the compounds of formula I may have 2 to 10 carbon atoms, preferably 2 to 6 carbon atoms and can be straight-chain or branched, saturated or unsaturated once or twice.

An acyl radical with 2 carbon atoms means, for example, an acetyl radical.

Examples of saturated, unbranched acyl radicals are an acetic acid residue, propionic acid residue, butyric acid residue, valeric acid residue, caproic acid residue, enanthic acid residue, caprylic acid residue, pelargonic acid residue and capric acid.

Examples of unbranched acyl residues which are unsaturated once are an acrylic acid residue and crotonic acid residue.

An example of an unbranched acyl radical which is unsaturated twice is a sorbic acid residue.

The alkyl radicals in the compounds of formula I may have from 1 to 6 carbon atoms and can be straight-chain or branched. Further, the alkyl radicals include saturated as well as unsaturated groups which latter groups contain one or two double bonds. Examples of alkyl radicals containing from 1 to 6 carbon atoms are methyl, ethyl, propyl, butyl, pentyl and hexyl, the n-isomers of all these radicals, isopropyl, isobutyl, 1-methylbutyl, isopentyl, neopentyl, 2,2-dimethylbutyl, 2-methylpentyl, 3-methylpentyl and isohexyl.

Unsaturated alkyl radicals are, for example, alkenyl residues such as vinyl, 1-propenyl, 2-propenyl (=allyl), 2-butenyl, 3-butenyl, 2-methyl-2-butenyl, 3-methyl-2-butenyl,5-hexenyl or 1,3-pentadienyl.

In one embodiment, the invention relates to the use of a compound of formula II

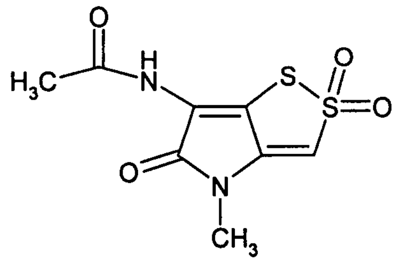

II

or a physiologically tolerated salt thereof in the manufacture of a medicament for the treatment of CNS disorders.

The compounds of formula I are useful in treating disorders caused by above normal levels of circulating neurolysin. CNS disorders which may be treated with the compounds of formula I include psychotic disorders such as schizophrenia and neurodegenerative disorders such as Alzheimer's and Parkinson's disease.

The compounds of formula I are obtainable by fermentation of the microorganism Nocardiopsis species ST 100692, DSM 13834, or one of its variants or mutants under suitable conditions, isolation of at least one compound, and conversion of the latter where appropriate into a physiologically tolerated salt, a derivative or a physiologically tolerated salt of the derivative.

The invention accordingly relates to a process for the production of a compound of formula I , which comprises cultivating the microorganism Nocardiopsis species ST 100692, DSM 13834, or mutants or variants thereof in an aqueous nutrient medium, isolating and purifying at least one target compound and optionally converting into a physiologically tolerated salt, a derivative or a physiologically tolerated salt of the derivative.

In addition to producing thiolutin dioxide, Nocardiopsis species ST 100692, DSM 13834, also produces thiolutin under the conditions of fermentation described. The thiolutin obtained may be isolated and converted into thiolutin dioxide by methods known to the skilled man.

An alternative process for the production of a compound of formula I according to the present invention accordingly comprises cultivating the microorganism Nocardiopsis species ST 100692, DSM 13834, or mutants or variants thereof, in an aqueous nutrient medium, isolating and purifying thiolutin, converting thiolutin into at least one target compound and optionally further converting into a physiologically tolerated salt, a derivative or a physiologically tolerated salt of the derivative.

The microorganism Nocardiopsis species ST 100692 was deposited on 13 November 2000 under the conditions of the Budapest treaty at the Deutsche Sammlung von Mikroorganismen und Zellkulturen, Mascheroder weg 1b, D-38124 Braunschweig under the number DSM 13834.

A taxonomic examination of the microorganism Nocardiopsis species ST 100692, DSM 13834, by analysis of fatty acids using gas chromatography showed the characteristic acids to be: 14:0 iso, 15:0 anteiso, 15:0 iso, 16: 0,16:0 iso, 17:0, 17:0 iso, 17:0 anteiso and 18:0. The colony colour is chrome yellow forming white aerial mycelium especially on ISP 2 (yeast-malt) and ISP 3 (oatmeal) medium.

In place of the strain DSM 13834 it is also possible to employ its mutants and variants so long as they synthesise the compounds according to the invention. Such mutants can be generated in a manner known per se by physical means, for example irradiation, such as, for example, ethyl methanesulfonate (EMS); 2-hydroxy-4-methoxybenzophenone (MOB) or N-methyl-N'-nitro-N-nitroguanidine (MNNG).

Screening for mutants and variants which produce the compounds according to the invention can take place by determining the biological activity of the active substances which have accumulated in the culture broth, for example by testing the neurolysin inhibition effect by the method described below.

Suitable and preferred as source of carbon for the aerobic fermentation are assimilable carbohydrates and sugar alcohols such as glucose, lactose or D-mannitol, and carbohydrate-containing natural products such as, for example, malt extract. Suitable nitrogen-containing nutrients are: amino acids, peptides and proteins, and their degradation products such as peptones or tryptones, also meat extracts, ground seeds, for example of corn, wheat, soybean or the cotton plant, distillation residues from the production of alcohol, meat meals or yeast extracts, but also ammonium salts and nitrates. Inorganic salts which the nutrient solution my contain are, for example, chlorides, carbonates, sulphates or phosphates of the alkali metals or alkaline earth metals, iron, zinc, cobalt and manganese.

The formation of thiolutin dioxide is achieved particularly well for example in a nutrient medium which contains about 0.5 to 5% glucose, preferably 1 to 2 %, 0.5 to 5 % soybean meal, preferably 1 to 2 %, 0.1 to 1.5 % corn steep (fluid), preferably 0.3 % to 0.8 %, 0.05 to 1.0 % calcium carbonate, preferably 0.1 to 0.5 % and 0.05 to 1 % sodium chloride, preferably 0.3 % to 0.8 %, in each case based on the weight of the complete nutrient solution.

The cultivation takes place aerobically, that is to say, for example, submerged with shaking or stirring in shaken flasks or fermenters, where appropriate with introduction of air or oxygen. The fermentation can be carried out, for

example, in wide-necked bottles or round-bottomed flasks of various volumes, in glass fermenters or stainless steel tanks. It can be carried out in temperature range of about 20 to 35°C preferably at about 25 to 30°C. The pH should be between 4 and 10, advantageously between 6 and 8. The microorganism is cultivated under these conditions in general for a period of from 20 to 200 hours, preferably 24 to 150 hours.

Cultivation is advantageously carried out in several stages, i.e. one or more precultures are initially produced in a liquid nutrient medium and are then transferred into the actual production medium, the main culture, for example in the ratio 1:10 by volume. The preculture is obtained, for example, by transferring a sporulated mycelium into the nutrient solution and allowing it to grow for about 20 to 120 hours, preferably 24 to 90 hours. The sporulated mycelium can be obtained, for example, by allowing the strain to grow for about 1 to 40 days, preferably 5 to 12 days, on a solid or liquid nutrient medium, for example yeast-malt agar or potato-dextrose agar.

The progress of the fermentation and the formation of the compounds according to the invention can be followed according to methods known to the skilled worker, such as, for example, by testing the biological activity in bioassays or by chromatographic methods such as thin-layer chromatography (TLC) or high performance liquid chromatography (HPLC).

The compounds of the formula I may occur both in the mycelium and in the culture filtrate, but the main amount is usually to be found in the biomass (mycelium). It is therefore expedient to separate the latter from the former by filtration or centrifugation. The filtrate is extracted with a water-immiscible solvent such as 1-butanol, ethyl acetate, chloroform or the like. The mycelium is expediently extracted with methanol or acetone, but it is also possible to use the above mentioned water-immiscible solvents.

The extractions can be carried out in a wide pH range, but it is expedient to operate in a neutral medium, preferably between pH 4 and pH 8. The organic extracts can, for example, be concentrated in vacuum and dried.

One purification method is chromatography on adsorption resins such as on Diaion® HP-20 (Mitsubishi Casei., Tokyo), on Amberlite ® XAD7 (Rohm and Haas, USA), and on Amberchrom® CG, (Toso Haas, Philadelphia, USA). Also suitable are numerous reverse phase supports, for example $RP_{18}$, as have become generally known, for example, within the framework of high pressure liquid chromatography (HPLC).

The compounds of the formula I can be isolated and purified in analogy to the methods described above or by other methods known to the skilled man.

Derivatives of thiolutin dioxide are also covered by formula I above. These derivatives have the same effect or are converted under mild conditions into compounds having the same effect as thiolutin dioxide. The derivatives may be prepared by processes well known to the skilled man. Examples of preparative processes of some of the thiolutin dioxide derivatives covered by formula I are given below:

1) The (exocyclic) acetyl group of thiolutin dioxide can be cleaved with acid or base as described in the literature, e.g. in 'Protective Groups in Organic Synthesis', 3rd Edition, T. Greene & P. Wuts, John Wiley & Sons, 1999, pp 553-555.

2) The free amino group of thiolutin dioxide can be alkylated, for example, via reductive alkylation as described in 'Advanced Organic Chemistry', 4th Edition, J. March, John Wiley & Sons, 1992, pp 898-900.

3) The amino group of thiolutin dioxide can be acylated, e.g. with acid chlorides or anhydrides by standard procedures well known to one skilled in the art.

Further derivatives of compounds of formula I include those compounds which result by a reduction of at least one double bond of a compound of formula I, such as thiolutin dioxide, by methods given in the literature, for example, in P. N. Rylander, 'Hydrogenation Methods', Academic Press, New York (1985), Chpt. 2 or by dehydrohalogenation by methods described in H. O. House in 'Modern Synthetic Reactions', W.A. Benjymin, Inc., New York (1972), pp 446-452.

The compounds according to the present invention may be converted into pharmaceutically acceptable salts. The salts can be prepared by standard procedures known to one skilled in the art.

Physiologically tolerated salts of the compounds of the formula I mean both the organic and the inorganic salts thereof as described in Remington's Pharmaceutical Sciences (17th edition, page 1418 (1985)). Salts like sodium and potassium salts, for example, may be prepared by treating the compounds according to the invention with suitable sodium or potassium bases.

A further aspect of the present invention is the use of a prodrug of a compound of formula I. Such a prodrug can be metabolised in vivo to a compound of formula I, such as thiolutin dioxide. These prodrugs may themselves be active or not.

The compounds of the formula I may exist in various polymorphous forms, for example as amorphous and crystalline polymorphous forms. All polymorphous forms of the compounds of the formula I fall within the scope of the invention and are a further aspect of the invention.

The compounds according to the present invention and their pharmaceutically acceptable salts and derivatives can be administered to animals, preferably to mammals, and in particular to humans as pharmaceuticals on their own or in mixtures with one another.

Suitable pharmaceutical compositions accordingly comprise an effective amount of one or more of the compounds of the formula I or II, or pharmaceutically acceptable salts thereof, together with a pharmaceutically acceptable carrier.

The compounds according to the invention can be administered orally, intramuscularly, intravenously or by other modes of administration. Pharmaceutical compositions which contain these compounds or a pharmaceutically acceptable salt or derivative thereof, optionally with other pharmaceutically active substances, can be prepared by mixing the active compounds with one or more pharmacologically tolerated auxiliaries and/or excipients. The mixture can then be converted into a suitable pharmaceutical form such as tablets, coated tablets, capsules, granules, powders, emulsions, suspensions or solutions.

Examples of auxiliaries and/or excipients which may be mentioned are fillers, emulsifiers, lubricants, masking flavours, colorants and buffer substances tragacanth, lactose, talc, agar-agar, polyglycols, ethanol and water. Suitable and preferred for parenteral administration are suspensions or solutions in water. It is also possible to administer the active substances as such, without vehicles or diluents, in a suitable form, for example, in capsules.

A method for the production of a suitable pharmaceutical comprises converting at least one of the compounds according to the present invention with a pharmaceutically suitable and physiologically tolerated carrier and, where appropriate, further suitable active substances, additives or excipients into a suitable dosage form.

As is customary, the galenic formulation and the method of administration as well as the dosage range which are suitable in a specific case depend on the species to be treated and on the state of the respective condition or disease, and can be optimised using methods known in the art. Using solid dosage forms, e.g. tablets or capsules, up to 500 mg, preferably 0.1 to 250 mg can be administered per day. For parenteral application up to 300 mg , preferably 0.5 to 150 mg, per day can be given.

The following are illustrative examples of the present invention but not limitative of the scope thereof:

Example 1: Production of a spore suspension of the producer strain Nocardiopsis species DSM 13834

100 ml of nutrient solution (4 g/l yeast extract, 15 g/l soluble starch, 1 g/l $K_2HPO_4$, 0.5 g/l $MgSO_4$ x 7 $H_2O$ in 1000 ml water, pH before sterilization 7.0) in a 300 ml sterile Erlenmeyer flask were innoculated with the strain Nocardiopsis species DSM 13834 and incubated at 28°C and 180 rpm on a rotary shaker for 5 days. Subsequently, 1.5 ml of this culture was diluted with 1.5 ml 99 % glycerin and stored at -20 °C.

Example 2: Production of a culture or of a pre-culture of the producer strain in an Erlenmeyer flask

A sterile Erlenmeyer flask containing 100 ml of the following nutrient solution: 15 g/l soya flour, 15 g/l glucose, 5 g/l corn steep fluid, 5 g/l NaCl and 2 g/l$CaCo_3$, was innoculated with a culture which had grown in a slant tube (same nutrient solution but with 2 % agar) or with 1 ml of a glycerin culture (see Example 1) and incubated on a shaker at 180 rpm and 25 °C. The maximum production of thiolutin dioxide was reached after about 96 hours. A 72- hour old submerged culture (produced according to the process described for the shake culture but with the following medium: 15 g/l glucose, 15 g/l soya flour, 5 g/l corn steep, 2 g/l $CaCo_3$ and 5 g/l NaCl, pH 7.5) was sufficient for inoculating 10 and 100 l fermenters with an inoculum of about 5 %.

Example 3: Production of thiolutin dioxide

A 200 l fermenter was operated under the following conditions:

| | | |
|---|---|---|
| Nutrient medium | Soya flour | 15 g/l |
| | Glucose | 15 g/l |
| | Corn steep | 5 g/l |
| | NaCl | 5 g/l |
| | $CaCO_3$ | 2 g/l |
| | pH 7.2 (before sterilization) | |
| Incubation time | 60 - 80 hours | |

(continued)

| | |
|---|---|
| Incubation temperature | 28 °C |
| Stirrer speed | 50 rpm |
| Aeration | 150 l/min |

Foaming was suppressed by a repeated addition of a few drops of 1 to 2 ml of ethanolic polyol solution. The production maximum was reached after 69 hours.

Example 4: Isolation of thiolutin dioxide

3 l of the culture solution obtained in Example 3 were lyophilised and the lyophilisate was subsequently extracted with methanol (2-3 l). The methanol extract was reduced in vacuum and diluted with water containing a methanol content of 10 %. The diluted extract was loaded onto a column with a capacity of 1 l packed with adsorption resin CHP-20P. Elution was with a solvent gradient from water to acetonitrile. Column through flow (30 ml/min) was carried out in fractions of 30 ml and the desired compound-containing fractions were collected, concentrated in vacuo and lyophilised to give approximately 30 mg of yellow-brown powder. The resultant powder was loaded onto a column packed with LUNA ® 10 uC18(2) (width x height = 21 mm x 250 mm) and eluted with a gradient of from 10 to 60 % acetonitrile in 0.1 % ammonium acetate/ water. The flow rate of the elution medium was 25 ml/min and outflow from the column was collected in fractions of 25 ml. Thiolutin dioxide was found in fractions 15 and 16. Lyophilisation of the named fractions gave 1.8 mg > 95 % pure thiolutin dioxide.

The physicochemical and spectroscopic properties of thiolutin dioxide can be summarised as follows:

Molecular formula: $C_8H_8N_2O_4S_2$
Molecular weight: 260.3
UV-Maxima: 230, 302, 388 nm
$^1$H- and $^{13}$-C NMR: see Table 1

Table 1:

| $^1$H- and $^{13}$-CNMR: Chemical shifts of thiolutin dioxide in DMSO at 300K | | |
|---|---|---|
| | $^1$H | $^{13}$C |
| 1 | 2.10 | 22.51 |
| 2 | - | 170.46 |
| 3 | 10.65 | - |
| 4 | - | 123.01 |
| 5 | - | 164.27 |
| 6 | 3.10 | 27.85 |
| 7 | - | 145.48 |
| 8 | - | 114.05 |
| 9 | 7.55 | 109.58 |

Example 5: Thiolutin dioxide as neurolysin inhibitor.

An assay was performed on a CyBio pipetting system in a 384-well plate format in a final assay volume of 16 µl.

In brief, 4 µl of appropriately diluted microbial extracts (dilution in 50 mM Tris buffer, pH 7.5) were distributed to the test plates (Greiner, white 384-small volume well plates). Thereafter, 4 µl of neurolysin (pre-diluted 1:5, 360 ng of protein) were added to each well. After a 10 minutes pre-incubation of the samples and the enzyme at room temperature the reaction was initiated by the addition of 8 µl of substrate (Mcc-Pro-Leu-Gly-D-Lys (Dnp)-OH, CALBIOCHEM) in Tris buffer. The final test concentration of the substrate was 4 µM.

After pipetting, the plates were immediately placed in a fluorometer (Spectra*Fluorplus*, SLT) and the initial amount of fluorescence was read ($\lambda_{ex}$: 360 nm / $\lambda_{em}$ 405 nm). The reaction was then allowed to proceed at 30°C for

30 min and a final fluorescence reading was taken.

The data are first blank corrected. Then, after subtracting the values at the time point zero from the respective values after 30 min, sample inhibition activity was expressed as

$$100 - (\text{Net Intensity}_{\text{compound}} / \text{Net Intensity}_{\text{control}}) \times 100\ (\%)$$

Each test plate contained a reasonable number of positive controls and blanks (buffer instead of enzyme).

The $IC_{50}$ of thiolutin dioxide was found to be 0.6 μM

**Claims**

**1.** The use of a compound of formula I

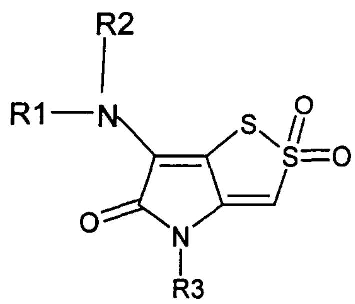

I

wherein
$R_1$, $R_2$ and $R_3$ are independently selected from: H, alkyl, and acyl, and the physiologically tolerated salts and derivatives thereof, in the manufacture of a medicament for the treatment of CNS disorders.

**2.** The use of a compound of the formula I as claimed in claim 1 wherein $R_1$ is acyl, $R_2$ is H and $R_3$ is alkyl.

**3.** The use according to claim 1 in which the compound is thiolutin dioxide of formula II

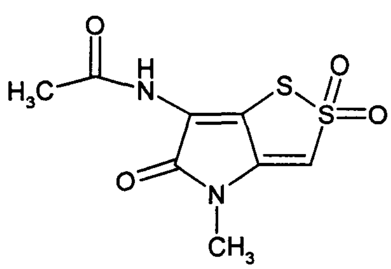

II

**4.** A process for the production of a compound of formula I, comprising cultivating the microorganism Nocardiopsis species ST 100692, DSM 13834, or mutants or variants thereof under aerobic conditions in a nutrient medium containing sources of carbon and nitrogen until at least one compound of formula I is present in the nutrient medium, and isolating and purifying the compound.

**5.** A process for the production of a compound of formula I , comprising cultivating the microorganism Nocardiopsis species ST 100692, DSM 13834, or mutants or variants thereof under aerobic conditions in a nutrient medium containing sources of carbon and nitrogen until thiolutin is present in the nutrient medium, isolating the thiolutin, and converting into at least one compound of formula I.

**6.** A process according to claim 4 or claim 5, further comprising converting said compound of formula I into a physiologically tolerated salt, derivative or physiologically tolerated salt of the derivative

**7.** Nocardiopsis species ST 100692 (DSM 13834).

European Patent Office

# EUROPEAN SEARCH REPORT

Application Number
EP 01 10 5959

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | WO 99 12543 A (CHEN GENHUI ;LI JIANXIONG (CA); WEBSTER JOHN MALCOLM (CA)) 18 March 1999 (1999-03-18) * claims 1-18 * --- | 1-3 | A61K31/407 A61P25/00 |
| A,D | WO 96 32396 A (WEBSTER JOHN MALCOLM ;LI JIANXIONG (CA); CHEN GENHUI (CA)) 17 October 1996 (1996-10-17) * claims 1-4 * ----- | 1-3 | |

TECHNICAL FIELDS SEARCHED (Int.Cl.7)
A61K

~~The present search report has been drawn up for all claims~~

| Place of search | Date of completion of the search | Examiner |
|---|---|---|
| MUNICH | 27 February 2002 | Trifilieff-Riolo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# LACK OF UNITY OF INVENTION
# SHEET B

Application Number
EP 01 10 5959

The Search Division considers that the present European patent application does not comply with the requirements of unity of invention and relates to several inventions or groups of inventions, namely:

1. Claims: 1-3

2. Claims: 4-7

## ANNEX TO THE EUROPEAN SEARCH REPORT ON EUROPEAN PATENT APPLICATION NO. EP 01 10 5959

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2002

| Patent document cited in search report | | Publication date | Patent family member(s) | | Publication date |
|---|---|---|---|---|---|
| WO 9912543 | A | 18-03-1999 | CA | 2212237 A1 | 05-03-1999 |
| | | | AU | 9057098 A | 29-03-1999 |
| | | | WO | 9912543 A1 | 18-03-1999 |
| | | | CN | 1276723 T | 13-12-2000 |
| | | | EP | 1009401 A1 | 21-06-2000 |
| | | | JP | 2001515861 T | 25-09-2001 |
| WO 9632396 | A | 17-10-1996 | AU | 5264796 A | 30-10-1996 |
| | | | CA | 2217957 A1 | 17-10-1996 |
| | | | WO | 9632396 A1 | 17-10-1996 |
| | | | US | 6316476 B1 | 13-11-2001 |

EPO FORM P0459

For more details about this annex : see Official Journal of the European Patent Office, No. 12/82